# EUROPEAN PATENT APPLICATION

(11) **EP 3 482 684 A1**
(43) Date of publication of application: **15.05.2019**
(21) Application number: 17200747.8
(22) Date of filing: 09.11.2017
(51) Int. Cl.: A61B 5/055, A61B 5/06, A61B 5/1455, A61B 34/20

(54) **BLOOD OXYGENATION MEASUREMENT AND DISPLAY IN MR-GUIDED CATHETERIZATIONS**

(71) Applicant: Koninklijke Philips N.V., 5656 AE Eindhoven (NL)
(72) Inventor: WEISS, Steffen, 5656 AE Eindhoven (NL)
(74) Representative: de Haan, Poul Erik

(57) **Abstract**

The present disclosure relates to a medical apparatus (126) comprising a display (132), a processor (130), and a memory (136) storing machine executable instructions (160-168). Execution of the instructions (160-168) causes the processor (130) to control the medical apparatus (126) to obtain a graphical representation (144) of magnetic resonance data (142) of an anatomical structure of interest (109). The graphical representation (144) is displayed on the display (132). Furthermore, a local blood oxygenation signal from a catheter (107) is received as well as a position tracking signal of the catheter (107) tracking positions (148) of the measurements of local blood oxygen saturation levels. Using the measured local blood oxygen saturation levels and the tracked positions (148) of the measurements, a spatial distribution (146, 402-408, 512-518, 612-618) of local blood oxygen saturation levels is displayed within the graphical representation (144).

## Description

### FIELD OF THE INVENTION

The invention relates to a medical apparatus, a computer program product and a method for displaying local blood oxygen saturation levels in magnetic resonance guided catheterization. More particular the invention relates to a medical apparatus, a computer program product and a method for displaying a spatial distribution of local blood oxygen saturation levels within a graphical representation of magnetic resonance images of an anatomical structure of interest.

### BACKGROUND OF THE INVENTION

Cardiac catheterizations are routinely performed e.g. in congenital heart patients to explore the hemodynamic state of their cardiovascular system and in particular their heart. Congenital heart diseases encompass a wide range of cardiac diseases, mostly associated with congenital anatomic malformations. An example of such a congenital heart disease are shunts which are frequent malformations representing anomalous short cuts between arterial and venous vessels. The detection, exact localization and treatment of such shunts are important goals of catheterizations.

In order to explore the hemodynamic state of a congenital heart patient, e.g. a right heart catheterization is performed. Such a right heart catheterization may be performed using a pulmonary artery catheters (PAC) that can measure the local intravascular blood pressure and blood oxygenation, also called Swan-Ganz-catheter, and provide clinically important information on cardiac performance and systemic oxygen transport. Right heart catheterizations are usually performed in catheterization laboratories under fluoroscopic guidance, i.e. X-ray guidance, e.g. in patients with congenital heart disease and/or pulmonary hypertension.

In current clinical practice flow-directed PACs are used under fluoroscopic guidance. However, ionizing radiation due to the fluoroscopic guidance may be problematic. In fluoroscopy, the patient must be exposed to a continuous source of X-rays instead of a momentary pulse which results in a significantly higher absorbed dose of radiation to which the patient is subjected. The potential risks from the absorbed dose has to be carefully balanced with the benefits of the procedure to the patient. In particular for pediatric or young adolescent patients which may often have to undergo several such fluoroscopic guided catheterization procedures, potential risks may outweigh the benefits.

Thus, there is a need for an alternative approach providing a visualization of the hemodynamic state of an anatomic structure of interest, like e.g. a heart, allowing to explore potential anomalies, e.g. caused by a congenital heart disease.

### SUMMARY OF THE INVENTION

In one aspect, the invention relates to a medical apparatus. The medical apparatus comprises a display, a processor for controlling the medical apparatus, and a memory storing machine executable instructions for execution by the processor. An execution of the instructions causes the processor to control the medical apparatus to:
- obtain a graphical representation of magnetic resonance data of an anatomical structure of interest;
- display the graphical representation on the display;
- receive a local blood oxygenation signal from a catheter providing local blood oxygen saturation levels measured by the catheter within the anatomical structure of interest;
- obtain a position tracking signal of the catheter tracking positions of the measurements of the local blood oxygen saturation levels within the anatomical structure of interest;
- display a spatial distribution of local blood oxygen saturation levels within the graphical representation displayed on the display using the measured local blood oxygen saturation levels and the tracked positions of the measurements.

The graphical representation images displayed on the display of the medical apparatus may comprise one or more magnetic resonance images or a segmentation of magnetic resonance images providing three-dimensional information about the anatomical structure of interest. This may be advantages to determine the distribution of local blood oxygen saturation levels in an anatomical structure of interest like a heart which is three dimensional and in which a catheter may be moved in three dimensions during a catheterization. For example, a segmentation may provide a continuous three-dimensional representation of a heart based on a three-dimensional set of magnetic resonance data. Alternatively or additionally, a plurality of two-dimensional magnetic resonance images depicting different cross-sections through the anatomical structure of interest may be displayed in order to provide a graphical representation which visualizes three-dimensional structural and hemodynamic information. The segmentation and/or magnetic resonance images may have been reconstructed from magnetic resonance data acquired by a magnetic resonance imaging system at the beginning of the catheterization session. The displayed graphical representation may for example comprise a three-dimensional representation of the heart of the patient or parts of the heart. In case of a segmentation, the graphical representation may show a mesh structure rendering the spatial contours of one or more segments of the anatomical structure of interest, like e.g. heart walls. The respective meshes may for example be constructed using a model-based segmentation of a three-dimensional magnetic resonance dataset.

Cardiac catheterization refers to an insertion of a catheter into a chamber or vessel of the heart. A catheter is a thin tube made from medical grade materials serving a broad range of functions and being configured for an insertion into a body cavity, duct, or vessel. Two major categories of cardiac catheterization may be differentiated: Left heart catheterization may e.g. allow for exploring the aorta, left ventricle, left atrium and pulmonary veins. Thus, blood oxygen saturation levels in the right side of the heart may be determined. For example, a thin, flexible wire may be inserted into either the femoral artery or the radial artery and threaded toward the heart until it is in the aorta. The wire may further be maneuvered into the left ventricle, left atrium and pulmonary veins. A catheter may be guided over the wire and enters either the left or right coronary artery. Right heart catheterization allows a determination of blood oxygen saturation levels within the right side of the heart. In this case, the heart may be accessed via the femoral vein. Measurements may be performed for the vena cava, right atrium right ventricle and pulmonary arteries.

The catheter may comprise a sensor for measuring the local blood oxygen saturation level within the anatomical structure of interest. When the catheter is moved through the respective anatomical structure of interest, like e. g. a blood vessel or a portion of a heart, the catheter measures the local blood oxygen saturation levels along its track through the anatomical structure of interest. In order to determine the positions of the measurements within the anatomical structure of interest, the position tracking signal of the catheter is used. The local blood oxygen saturation levels measured may be displayed within the graphical representation on the display. In addition, interpolated or extrapolated local blood oxygen saturation levels may be displayed on order provide a continues illustration of distribution of local blood oxygen saturation levels in two or three dimensions based on the local blood oxygen saturation levels measured along the track of the catheter. The local blood oxygen saturation levels may for example be displayed in form of a color coding of the anatomical structure depicted by the graphical representation. Thus, a spatial distribution of the local blood oxygen saturation levels is generated and displayed.

The local oxygen saturation level refers to a local fraction of oxygen-saturated hemoglobin relative to total hemoglobin, i.e. unsaturated as well as saturated, locally present in the blood. The human body requires and regulates a rather precise and specific balance of oxygen in the blood. Normal local oxygen saturation level levels in humans are considered 95-100 percent. If the level drops below 90 percent, it is considered low resulting in hypoxemia. Blood oxygen levels below 80 percent may compromise organ function, e.g. the heart, and may have to be addressed promptly. Continued low oxygen levels may lead to respiratory or cardiac arrest.

According to embodiments, at the beginning of the catheterization session, a three-dimensional magnetic resonance dataset of the anatomical structure of interest which is to be examined may be acquired and a graphical representation be generated using the acquired dataset. The graphical representation is displayed in form of one or more magnetic resonance images and/or a segmentation of the anatomical structure of interest. When the catheter is moved through the respective anatomical structure of interest, not only the position of the respective catheter may be displayed within the graphical representation by adding a position indicator indicating, but also the spatial distribution of the blood oxygen saturation levels. The displayed visualization of the position of the catheter as well as the spatial distribution of the blood oxygen saturation levels may continuously be updated using newly received data of the current position and local blood oxygen saturation level in real-time during the catheterization procedure. As the catheter is moved forward, the spatial distribution display extends further and further through the displayed three-dimensional representation. For example, a color-coding may be used to display measured and/or interpolated/extrapolated local blood oxygen saturation levels.

Embodiments may have the beneficial effect of enabling an MR-guided catheterization. The catheterization is MR-guided using the graphical representation within which the position of the measurement of the local blood oxygen saturation level, the position of the catheter and/or the orientation of the catheter are indicated relative to the anatomical structure of interest. An actively tracked catheter, e.g. MR-tracked or tracked using an electro-magnetic tracking system, may be used to enable real-time 3D localization of the position of the measurement of the local blood oxygen saturation level, the position of the catheter and/or the orientation of the catheter.

MR-guidance may have a number of beneficial effect, like e.g. an avoidance of ionizing radiation especially in pediatric or young adolescent patients that often have to undergo several procedures. In current clinical practice, flow-directed PACs are used under fluoroscopy guidance, which are constructed such that the blood flow can easily advance a tip balloon fixed to a highly flexible catheter tube into the pulmonary artery in anatomically normal hearts. Malformations of the heart as shunts, vascular narrowing, or valve deformations may compromise this approach making it difficult to have the catheter advanced in a controlled way to various locations within the heart. According to embodiments, an actively tracked and actively steered PAC may be used for the MR-guided catheterizations. Such embodiments may allow to navigate the catheter with full control even in patients with congenital malformations.

Active catheter tracking may provide the catheter position in real-time in relation to 3D MRI data of the anatomical structure of interest, like e.g. cardiac anatomy, depicted by the graphical representation on the display. It is proposed to associate the catheter position data to the oximetry data, i.e. the local blood oxygen saturation levels, measured by the catheter as well as to the anatomical structure of interest, e.g. the cardiac anatomy, imaged by a magnetic resonance imaging system. Thus, a 3D map of blood oxygenation in form of the spatial distribution of local blood oxygen saturation levels throughout the anatomical structure of interest, e.g. the chambers of the heart, maybe provided.

In conventional fluoroscopy guided catheterization procedures, oximetry data may only be displayed as a percent value and a time curve, because the position of the catheter tip is not measured in 3D but only visible on the fluoroscopy display. A relation between the oximetry data and position data is neither determined nor displayed. In addition, fluoroscopy images do not display structures in 3D, like e.g. cardiac structures, which may be important to understand the structure of malformations such as shunts.

According to embodiments, a continuous measurement of local blood oxygenation is applied together with continuous catheter tracking, e.g. using MR-tracking or an electro-magnetic tracking system, to acquire a 3D spatial distribution of local blood oxygen saturation levels, i.e. a 3D map of blood oxygenation, throughout an anatomical structure of interest, like e.g. one or more chambers of a heart. The spatial distribution may be displayed as color-coding within the graphical representation. The color-coding maybe displayed on one or more contours of the anatomical structure of interest, like e.g. on the endo-cardinal surface of the heart chambers. The respective contours may be rendered in 2D or 3D using a segmentation of a 2D or 3D MR dataset acquired at the beginning of the intervention.

According to embodiments, an additional set of magnetic resonance data may be acquired during the intervention in order to update the displayed graphical representation.

Embodiments may have the beneficial effect of providing an intuitive visualization of the oxygenation state of the anatomical structure of interested and enabling quick detection of deviations from the normal oxygenation e.g. caused by vascular shunts. Once such a shunt is suspected, its exact location may be readily explored with the catheter because the oxygenation color-coding is updated on-the-fly.

Embodiments may further have the beneficial effect that they provide a detailed image of the anatomical structure to be examined using the magnetic resonance data, represented e.g. by one or more magnetic resonance image or a segmentation. Furthermore, the examiner is provided with information about the position and/or orientation of the catheter used for the examination. In addition, the spatial distribution of local blood oxygen saturation levels within the anatomical structure of interest is presented, such that the examiner can determine a presence and/or position of anomalies within the anatomical structure of interest regarding the local blood oxygen saturation level. Based on the position information, the examiner may further examine the anatomical structure at the respective position and determine the anatomical source of the detected anomaly.

According to embodiments, the spatial distribution of local blood oxygen saturation levels is displayed within the anatomical structure of interest depicted by the graphical representation. According to embodiments, the displayed spatial distribution of local blood oxygen saturation levels comprises the measured local blood oxygen saturation levels. According to embodiments, the displayed spatial distribution of local blood oxygen saturation levels comprises local blood oxygen saturation levels interpolated or extrapolated from the measured local blood oxygen saturation levels. According to embodiments, the displayed spatial distribution of local blood oxygen saturation levels comprises only local blood oxygen saturation levels extrapolated or interpolated from the measured local blood oxygen saturation levels.

The obtaining of the graphical representation of magnetic resonance data may comprise acquiring magnetic resonance imaging data and reconstructing magnetic resonance images and/or perform a segmentation by the medical apparatus. For example, the medical apparatus may comprise the magnetic resonance imaging system used for acquiring the magnetic resonance data. In a further example, the obtaining of the graphical representation may comprise accessing a storage device and reading from the storage device the respective representation. In another example, the respective representation may be received from a magnetic resonance imaging system generating the graphical representation or from a computer system with access to a storage device comprising the respective representation. The graphical representation may be received upon a request by the medical apparatus sent to the respective magnetic resonance imaging system or the respective computer system. In a further example, the obtaining of the graphical representation may be performed automatically.

According to embodiments, the graphical representation depicts at least portions of the anatomical structure of interest extending in three orthogonal directions in space. Embodiments may have the beneficial effect of providing a three-dimensional representation of at least portions of the anatomical structure of interest. According to embodiments, the graphical representation comprises a segmentation depicting one or more segments of the anatomical structure of interest and/or one or more magnetic resonance images, each magnetic resonance image depicting a cross-section through the anatomical structure of interest.

According to embodiments, the graphical representation displayed on the display comprise one or more two-dimensional magnetic resonance images depicting a cross section through the anatomical structure of interest and/or portions of the anatomical structure of interest. According to embodiments, two such two-dimensional magnetic resonance images are displayed. According to embodiments, the two two-dimensional magnetic resonance images depict two orthogonal cross sections through the anatomical structure of interest and/or portions of the anatomical structure of interest. According to embodiments, the two two-dimensional magnetic resonance images intersect at a common line.

According to embodiments, three such two-dimensional magnetic resonance images are displayed. According to embodiments, the three two-dimensional magnetic resonance images depict three orthogonal cross sections through the anatomical structure of interest and/or portions of the anatomical structure of interest. According to embodiments, the three two-dimensional magnetic resonance images intersect at a common point.

According to embodiments, the graphical representation depicts rendered spatial contours of one or more three-dimensional segments of the anatomical structure of interest and/or portions of the anatomical structure of interest. According to embodiments, the spatial contours are rendered by one or more polygonal meshes.

Segmentation may be used to identify contours of the anatomical structure of interest. Different segmentation protocols maybe used. For rendering the contours of the anatomical structure of interest, the segmentation protocol may comprise an auto-contouring program logic configured for automatically identifying the contour of anatomical structures within magnetic resonance images may be used.

Embodiments may have the beneficial effect that applying an auto-contouring program logic for automatically identifying the contours of the anatomical structure of interest may allow for an automatization of the displaying process. In particular, the image processing may be sped up compared to manual methods and may provide accurate and reliable results. An auto-contouring program logic may for example be based on a shape-constraint deformable model of the anatomical structure of interest. For example, the anatomical structure of interest may be a heart. The deformable model may be provided in form of a surface mesh resembling the surface geometry of the respective anatomical structure of interest including a plurality of polygons, each polygon further including vertices and edges. For example, the polygons may have triangular shapes including three vertices and edges each. The model may automatically be fitted to the line structure shown by the magnetic resonance images. Thus, the contours of the respective anatomical structure of interest may be identified and rendered. For example, the model may be a three-dimensional model and the contour lines identified may result from an intersection of the three-dimensional model with a plurality of sectional planes, e.g. parallel sectional panes, through the respective anatomical structure of interest.

According to embodiments, the anatomical structure of interest comprises left atrium of a heart, right atrium of the heart, left ventricle of the heart, right ventricle of the heart and/or one or more blood vessels, like e.g. vena cava, aorta, pulmonary veins or pulmonary arteries.

According to embodiments, the displaying of the spatial distribution of the local blood oxygen saturation levels comprises interpolating or extrapolating additional local blood oxygen saturation levels at positions within the anatomical structure of interest, for which no measured local blood oxygen saturation levels are provided by the catheter, using the measured local blood oxygen saturation levels and the tracked positions of the measurements, the displayed spatial distribution of the local blood oxygen saturation levels comprising the additional local blood oxygen saturation levels.

As the catheter may provide only local blood oxygenation saturation levels, embodiments may have the beneficial effect that they enable an extension of the spatial distribution of the local blood oxygenation saturation levels along the track of the catheter through the anatomical structure of interest. For example, the spatial distribution of the local blood oxygen saturation levels may be interpolated and/or extrapolated throughout the inner space defined by the displayed contours. Thus, the examiner is enabled to determine possible anomalies of the local blood oxygen saturation levels beyond the track of the catheter through the anatomical structure of interest. For example, an extrapolation of the local blood oxygen saturation levels may indicate that there is an anomalous rise or fall of the respective levels at one side of the track of the catheter. The examiner may thus be enabled to deviate from the planned track of the catheter and to follow the gradient of the local blood oxygen saturation levels to the source of the anomaly.

According to embodiments, for the interpolating or the extrapolating of each of the additional local blood oxygen saturation levels only those measured local blood oxygen saturation levels are taken into account at positions which are geometrically connectable with the position at which the respective additional local blood oxygen saturation level is to be interpolated or extrapolated by a line such that the line is not crossing any spatial contour of the anatomical structure of interest and which have a distance along the respective line from the position at which the respective additional local blood oxygen saturation level is to be interpolated or extrapolated that is smaller than a predefined maximum distance.

Embodiments may have the beneficial effect that for the interpolation or extrapolation only those measured local blood oxygen saturation levels are taken into account which are not separated by a spatial contour of the anatomical structure of interest. Considering a blood vessel or a heart, local blood oxygen saturation levels may only influence each other in case a fluid passage connecting the positions of the respective local blood oxygen saturation levels, i.e. if a fluid connection exists. In case there is no fluid passage, i.e. the two positions are isolated from each other such that no fluid can be exchanged between the two positions, they cannot influence each other.

Embodiments may further have the beneficial effect of taking into account the actual distance which the blood has to cover in order to get from one position within the anatomical structure of interest to another, i.e. along the respective line not crossing any spatial contours. Even though positions may spatially be near each other, the distance has to cover to get from one to the other may be significant such that any mutual influence between the same may be excluded.

According to embodiments, oxygenation data, i.e. local blood oxygen saturation levels, may be interpolated or extrapolated in-between or around positions at which oxygenation data has been measured by the catheter. For the interpolation and/or extrapolation, known methods may be used, like e.g. radial basis functions. Furthermore, the knowledge of the spatial contours of the anatomical structure of interest, e.g. cardiac structures, may be exploited to improve both interpolation and extrapolation. For example, only selected measured data may used to perform the interpolation or extrapolation at a given point in space. This may important if using a known method for interpolation or extrapolation such as e.g. a method based on radial basis functions, which usually have Gauss functions as kernels that may be non-zero also in other portions of the anatomical structure of interest, like e.g. in other cardiac chambers. Without any explicit data point selection, an oxygenation measurement at a point in the left ventricle next to the septum could strongly contribute to the interpolation of values near-by in the right ventricle, which does not make any sense in terms of physiology, because the septum is in-between separating the fluid dynamics of the left ventricle from the fluid dynamics of the right ventricle.

According to embodiments, for the interpolation or extrapolation at a point x only those measured data points yᵢ may be selected to contribute to the interpolation or extrapolation for which a line from x to yᵢ does not cross any cardiac wall structure and the distance between x and yᵢ along the respective line is smaller than a predefined threshold. A fluid exchange may only appear along such continuous lines not crossing any cardiac wall structure. However, only measured data points yᵢ which are not too far away in terms of distance along such lines of fluid exchange may provide a non- negligible influence on the interpolation or extrapolation at a point x. Furthermore, the direction of blood flow may be taken into account. For example, the predefined threshold for the distance between x and yᵢ along the respective line may be larger in case yᵢ is located against the direction of blood flow from the point of view of x, i.e. blood is flowing from yᵢ to x. In case yᵢ is located in the direction of blood flow from the point of view of x, i.e. blood is flowing from x to yᵢ, the predefined threshold for the distance between x and yᵢ along the respective line may be smaller. Effectively, only those points yᵢ may contribute to the interpolation or extrapolation that are located inside the same heart chamber as x. Other points yᵢ' hidden behind cardiac walls may be disregarded.

According to embodiments, in a first display mode, the displayed graphical representation comprises one or more of the two-dimensional images generated using multiplanar reformation. The spatial distribution of the local blood oxygen saturation levels is interpolated or extrapolated in the respective one or more two-dimensional images.

The spatial distribution of the local blood oxygen saturation levels may for example be interpolated or extrapolated throughout one, two or three planes. In case of more than one plane, the respective planes may be orthogonal to each other. The one or more planes may follow the track of the catheter through the anatomical structure of interest. In addition, the one or more planes may automatically be oriented such that the tip section of the catheter is comprised by the imaging planes represented by the images. Embodiments may have the beneficial effect that the plane two-dimensional images of cross-sections through the anatomical structure of interest may comprise the current position tracked by the tracking signal, i.e. current measurement of the local blood oxygen saturation levels.

According to embodiments, in the first display mode the spatial distribution of the local blood oxygen saturation levels is interpolated or extrapolated in three orthogonal planes through a common center point which automatically follows the positions tracked by the tracking signal of the catheter.

According to embodiments, the spatial distribution of the local blood oxygen saturation levels is displayed in form of an extrapolation of the respective levels onto on one or more spatial contours of the anatomical structure of interest depicted by the one or more magnetic resonance images.

According to embodiments, in a second display mode, the displayed graphical representation comprises the segmentation depicting one or more segments of the anatomical structure and the displayed spatial distribution of the local blood oxygen saturation levels comprises additional local blood oxygen saturation levels extrapolated on one or more spatial contours of the one or more segments.

Embodiments may have the beneficial effect that the local blood oxygen saturation levels on the spatial contours, i.e. surfaces of the respective contour, are displayed. Since structural anomalies of the respective contours are most likely the source of potential anomalies of the local blood oxygen saturation levels, displaying the blood oxygen saturation levels on the spatial contours may link the local blood oxygen saturation levels to positions on the spatial contours, which may be further examined in order to determine anomalies of the respective spatial contours.

According to embodiments only local blood oxygen saturation levels extrapolated on the spatial contours of the one or more segments are displayed. Embodiments may have the beneficial effect that the amount of displayed data is reduced. This may facilitate the understanding of the displayed information by the examiner as well as reduce the workload for the processor in order to calculate the information necessary for displaying the spatial distribution of the local blood oxygen saturation levels. This may be particularly relevant in case the spatial distribution is continuously updated.

According to embodiments, the interpolation or extrapolation of the additional local blood oxygen saturation levels comprised by the displayed spatial distribution of local blood oxygen saturation levels are continuously updated using the measured local blood oxygen saturation level currently received.

Embodiments may have the beneficial effect of providing continuously updated spatial distribution of the local blood oxygen saturation levels. Each additional measured local blood oxygen saturation level may be used to update the displayed spatial distribution. Thus, as the catheter is moved through the anatomical structure of interest more and more precise representation of the spatial distribution of local blood oxygen saturation levels is displayed.

According to embodiments, the spatial distribution of the local blood oxygen saturation levels is displayed in form of a line following the tracked positions of the measurements of the local blood oxygen saturation levels within anatomic structure of interest depicting the local blood oxygen saturation levels measured at the respective tracked positions.

Embodiments may have the beneficial effect that they provide a fast and simple line representation of the spatial distribution of the local blood oxygen saturation levels through the anatomical structure of interest. Such a simplified spatial distribution, i.e. one-dimensional representation of the spatial distribution, may be used for a determination whether there are anomalies present and/or to identify regions of the anatomical structure of interest, wherein anomalies may potentially be present and which may have to be more in detail.

According to embodiments, the displaying of the spatial distribution of the local blood oxygen saturation levels further comprises providing an alarm signal if one of the local blood oxygen saturation levels comprised by the spatial distribution of local blood oxygen saturation levels or if a spatial and/or temporal gradient of the local blood oxygen saturation levels exceeds a threshold. The alarm signal may e.g. be displayed on the display. The alarm may e.g. highlight the local blood oxygen saturation level or the gradient exceeding the threshold. An anomalous high or low local blood oxygen saturation level as well as an anomalous high spatial and/or temporal change of the local blood oxygen saturation levels may indicate a physiological anomaly.

According to embodiments, the alarm may comprise switching from the first display mode to the second display mode. Embodiments may have the beneficial effect that the first display mode may be more advantageous for providing a representation of the spatial distribution of the local blood oxygen saturation levels throughout the anatomical structure of interest in form of cross-sections through the entire anatomical structure. The second display mode may be advantageous for identifying anomalies of the structure of the contours of the anatomical structure of interest.

According to embodiments, the medical apparatus further comprises a magnetic resonance imaging system for acquiring magnetic resonance data from an imaging zone. The execution of the instructions further causes the processor to control the magnetic resonance imaging system to:
- acquire magnetic resonance data of the anatomical structure of interest within the imaging zone;
- reconstruct the graphical representation using the acquired magnetic resonance data;
- provide the graphical representation.

Embodiments may have the beneficial effect that the present medical apparatus may use and/or be integrated into an existing MRI system. MRI may have the advantage that it is particularly useful for scanning and detecting abnormalities in soft tissue structures like, e.g. the heart. No radiation dangerous for the patient is involved in MRI image acquisition, such that it does not negatively affect the body. In addition, MRI scans may provide information about the blood circulation throughout the body and blood vessels and also enable the detection of problems related to the blood circulation.

According to embodiments, the reconstruction of the three-dimensional image comprises:
- reconstructing three-dimensional image data from the acquired magnetic resonance data,
- determining one or more three-dimensional segments of the three-dimensional image data corresponding to the anatomical structure of interest and/or a portion of the anatomical structure of interest, in which the catheter measures the local blood oxygen saturation levels.
- rendering the spatial contours of the one or more segments.

Embodiments may have the beneficial effect of providing spatial contours of the structure of interest. In particular, considering blood vessels and/or portions of the human heart as the anatomical structure of interest, the respective structures are provided by walls which form the contours and define an inner space filled with blood. The catheter is moved through the respective inner space and a spatial distribution of the local blood oxygen saturation levels within the same is determined. Thus, the contours on the one hand provide a valuable help for the spatial orientation. On the other hand, the contours identify the portions of the respective anatomical structure of interest which are most likely responsible for the local blood oxygen saturation levels. Thus, the displayed contours provide a realistic image of the main portions of the anatomical structure to be examined.

According to embodiments, the medical apparatus further comprises the catheter. The execution of the instructions further causes the processor to control the medical apparatus to:
- provide the local blood oxygenation signal;
- provide the position tracking signal of the catheter.

Local blood oxygen saturation levels may e.g. be continuously measured using fiberoptic reflection spectrophotometry. Light is emitted from a light source, like e.g. an LED, located in an oximetry device that is attached to the distal end of the catheter through a first fiberoptic channel comprised by the catheter into the blood, e.g. venous blood, at the tip of the catheter. Some of this light is reflected back and received by a second fiberoptic channel comprised by the catheter, which is read by a photodetector located in the oximetry device. The respective oximetry device may be provided in addition to the medical apparatus or comprised by the medical apparatus. The amount and spectrum of light that is reflected by the blood is determined by the oxygen saturation of hemoglobin. This information is processed by the oximetry device and updated and displayed, e.g. at typical rate of 0.5 Hz, as a percent value.

According to embodiments, the position tracking signal of the catheter is received from the catheter. According to embodiments, the catheter is configured for active position tracking. According to embodiments, the position is actively tracked using the magnetic resonance imaging system. According to embodiments, the position is actively tracked using an electron-magnetic system. According to embodiments, the position tracking signal of the catheter further tracks the orientation of the catheter within the anatomical structure of interest.

According to embodiments, the catheter is configured to be actively steered. Active steering may allow to navigate the catheter with full control even in patients with congenital malformations. Alternatively, a simple version of the proposed catheter be used which only provides active tracking but not active catheter steering. This flow directed version may be sufficient to be used in patients with only minor malformations. However, the full potential of actively exploring shunts in a procedure may be effectively leveraged with an actively steerable catheter.

According to embodiments the position tracking signal of the catheter may be obtained using a magnetic resonance imaging system. In order to track the position of the catheter, active magnetic resonance tracking of the catheter may be used. In case of active a magnetic resonance tracking of the catheter, the catheter may comprise one or more coils located along its longitudinal extension. The respective coils detect a local magnetic field strength of a spatially varying magnetic field generated by the magnetic resonance imaging system. Based on the local magnetic field strength, the position of the respective coil within the magnetic resonance field can be determined. Using the magnetic resonance imaging system for determining the position of the catheter may have the beneficial effect that the position of the catheter using the same frame of reference used for generating the graphical representation displayed on the display. Thus, it may not be necessary to register the position tracking signal and the imaging signals. This may have the beneficial effect of saving time and simplifying the data evaluation.

In case an additional system, like e.g. an electro-magnetic tracking system is used for tracking the position of the catheter, registration may be necessary, since the magnetic resonance imaging system and the position tracking system may use different spatial frames of reference. A registration may in general refer to alignment between the position signal and the graphical representation. A registration may for example be performed in a deformable or rigid form. A rigid registration uses a simple transformation which is uniformly applied to the position signal and/or the graphical representation. A deformable registration allows a non-uniform mapping between the position signal and the graphical representation.

According to embodiments an electro-magnetic tracking system may be provided in order to track the position of the catheter. The electro-magnetic tracking system may comprise a plurality of coils generating varying electro-magnetic fields combining to a spatially varying electro-magnetic field. The catheter may comprise one or more coils configured for determining a local field strength of the spatially varying electro-magnetic field generated by the electro-magnetic tracking system. The local electro-magnetic field strength determined by the one or more coils of the catheter, and the position and/or orientation of the catheter within the spatially varying electro-magnetic field generated by the electro-magnetic tracking system may be determined. For example, voltage fluctuations induced in the catheter due to moving the catheter within the varying magnetic field may be measured and evaluated in order to determine the position and/or orientation of the catheter within the respective varying magnetic field.

In another aspect, the invention relates to a computer program product. The computer program product comprises machine executable instructions for execution by a processor controlling a medical apparatus. The medical apparatus further comprises a display and a memory for storing the machine executable instructions for execution by the processor. An execution of the instructions causes the processor to control the medical apparatus to:
- obtain a graphical representation of magnetic resonance data of an anatomical structure of interest;
- display the graphical representation on the display;
- receive a local blood oxygenation signal from a catheter providing local blood oxygen saturation levels measured by the catheter within the anatomical structure of interest;
- obtain a position tracking signal of the catheter tracking positions of the measurements of the local blood oxygen saturation levels within the anatomical structure of interest;
- display a spatial distribution of local blood oxygen saturation levels within the graphical representation displayed on the display using the measured local blood oxygen saturation levels and the tracked positions of the measurements.

In another aspect, the invention relates to a method for operating a medical apparatus. The medical apparatus comprises a display, a processor for controlling the medical apparatus, and a memory storing machine executable instructions for execution by the processor. An execution of the instructions causes the processor to control the medical apparatus to execute the method comprising:
- obtaining a graphical representation of magnetic resonance data of an anatomical structure of interest;
- displaying the graphical representation on the display;
- receiving a local blood oxygenation signal from a catheter providing local blood oxygen saturation levels measured by the catheter within the anatomical structure of interest;
- obtaining a position tracking signal of the catheter tracking positions of the measurements of the local blood oxygen saturation levels within the anatomical structure of interest;
- displaying a spatial distribution of local blood oxygen saturation levels within the graphical representation displayed on the display using the measured local blood oxygen saturation levels and the tracked positions of the measurements.

It is understood that one or more of the aforementioned embodiments of the invention may be combined as long as the combined embodiments are not mutually exclusive. Ordinal numbers, like e.g. 'first' and 'second', are used herein to indicate different element assigned with the same name, but not to establish any order of the respective elements.

### BRIEF DESCRIPTION OF THE DRAWINGS

In the following preferred embodiments of the invention will be described, by way of example only, and with reference to the drawings in which:
Fig. 1 depicts a schematic block diagram of an exemplary medical apparatus,
Fig. 2 depicts a schematic block diagram of an exemplary medical apparatus comprising a magnetic resonance imaging system and a catheter,
Fig. 3 depicts a schematic block diagram of an exemplary catheter,
Fig. 4 depicts a schematic diagram of a heart illustrating exemplary local blood oxygen saturation levels,
Fig. 5 depicts a schematic diagram of a graphical representation showing a mesh structure rendering spatial contours of a heart,
Fig. 6 depicts a schematic diagram of a graphical representation showing a mesh structure rendering spatial contours of a right atrium,
Figs. 7 depict schematic diagrams of graphical representations comprising two-dimensional magnetic resonance images,
Fig. 8 depicts a schematic flowchart of an exemplary method for using the medical apparatus.

### DETAILED DESCRIPTION OF THE EMBODIMENTS

In the following, like numbered elements in the figures are either similar elements or perform an equivalent function. Elements which have been discussed previously will not necessarily be discussed in later figures if the function is equivalent.

Various structures, systems and devices are schematically depicted in the figures for purposes of explanation only and so as to not obscure the present invention with details that are well known to those skilled in the art. Nevertheless, the attached figures are included to describe and explain illustrative examples of the disclosed subject matter.

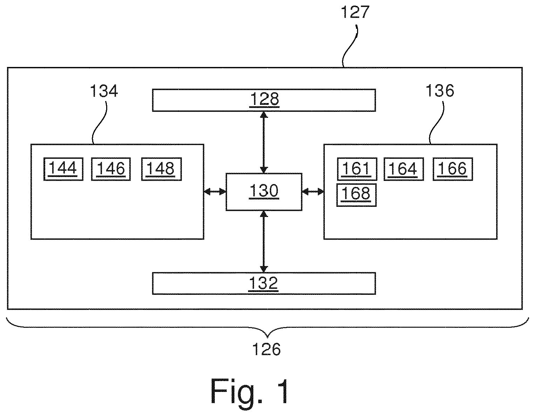

Fig. 1 illustrates an exemplary medical apparatus 126 for displaying a spatial distribution of local blood oxygen saturation levels within a graphical representation of an anatomical structure of interest using local blood oxygen saturation levels measured by a catheter and tracked positions of the respective measurements. Medical apparatus 126 furthermore provides MRI-based guidance for the catherization executed with the catheter in order to measure the local blood oxygen saturation levels. The medical apparatus 126 may comprise a computer system 127. The computer 127 is shown as containing a processor 130 which is operable for executing machine-readable instructions. The computer 127 is further shown as comprising a user interface 132, computer storage 134 and computer memory 136 which are all accessible and connected to the processor 130. The user interface 132 may provide a graphical user interface in form of a display for displaying a graphical representation of an anatomical structure of interest. The medical apparatus 126 may be configured to receive via the communication interface 128 magnetic resonance imaging data and/or a graphical representation of magnetic resonance data of an anatomical structure of interest. The respective graphical representation may e.g. be received from a magnetic resonance imaging system or a database. According to embodiments, magnetic resonance imaging data is received and used by the medical apparatus 126 to reconstruct a graphical representation, e.g. one or more magnetic resonance images or a segmentation, which is displayed on the display provided by the user interface 132.

The medical apparatus 126 may further be configured to receive via the communication interface 128 a local blood oxygenation signal from a catheter and a position tracking signal of the catheter, e.g. from the catheter. The position tracking signal may be generated using a magnetic resonance tracking system, e.g. the magnetic resonance imaging system tracking, or an electro-magnetic position tracking system. According to embodiments the database and/or the magnetic resonance imaging system may be comprised by the medical apparatus 126.

The computer storage 136 is shown as containing a graphical representation 144 of an anatomical structure of interest to be displayed on the display. In addition, the computer storage 136 comprises a spatial distribution of local blood oxygen saturation levels 146 to be displayed within the graphical representation 144, e.g. as a color-coding. Furthermore, the computer storage 136 comprises data 148 about the position and/or orientation of the catheter within the anatomical structure of interest. The data 148 being configured to display the position and/or orientation of the catheter within the anatomical structure of interest depicted by the graphical representation 144.

The computer memory 136 is shown as comprising a control module 161. The control module 161 contains computer executable code or instructions which enable the processor 130 to control the operation and function of the medical apparatus 126.

The computer memory 138 may further contain a spatial distribution generating module 164. The spatial distribution generating module 164 contains computer executable code or instructions which enable the processor 130 to generate the spatial distribution of local blood oxygen saturation levels 146 using the measured local blood oxygen saturation levels and the tracked positions of the measurements. The spatial distribution generating module 164 may e.g. perform interpolations and/or extrapolations in order to generate the spatial distribution of local blood oxygen saturation levels 146.

Furthermore, the computer memory 138 may comprise a catheter position generating module 166 containing computer executable code or instructions which enable the processor 130 to generate data 148 identifying the position and/or orientation of the catheter within the anatomical structure of interest depicted by the one or more magnetic resonance images 144.

Finally, computer memory 138 may comprise a display control module 168 containing computer executable code or instructions. The computer executable code or instructions of the display control module 168 enable the processor 130 to display the graphical representation 144, the spatial distribution of local blood oxygen saturation levels 146 and the position as well as the orientation of the catheter 148 on the display provided by the user interface 132.

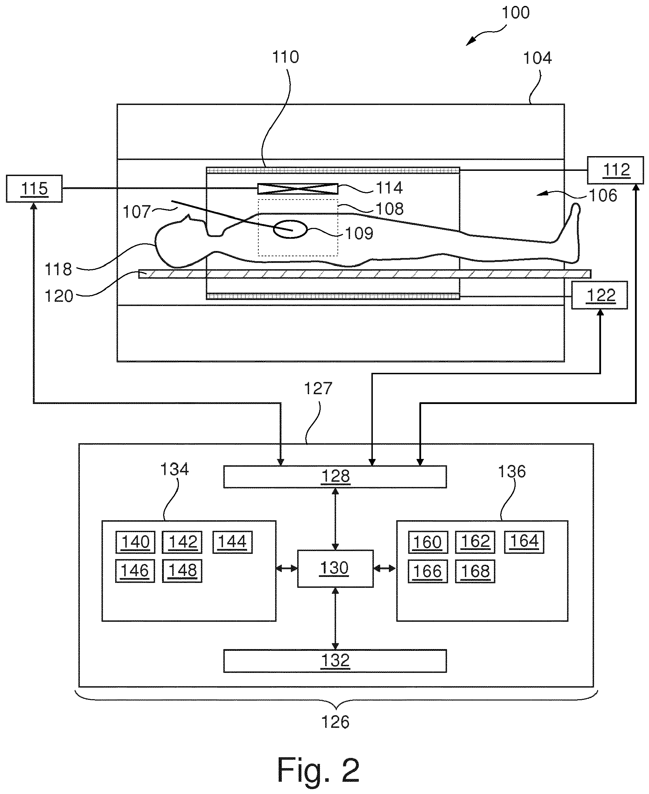

Fig. 2 illustrates an exemplary medical apparatus 126 which corresponds to the medical apparatus 126 of Fig. 1 and in addition to the computer 127 further comprises a magnetic imaging system 100 as well as a catheter 107. The magnetic resonance imaging system 100 comprises a main magnet 104. The main magnet 104 is a superconducting cylindrical type magnet 104 with a bore 106 through it. The use of different types of magnets is also possible. For instance, it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 106 of the cylindrical magnet 104 there is an imaging zone 108 where the magnetic field is strong and uniform enough to perform magnetic resonance imaging. Within the imaging zone 108 an anatomical structure of interest 109, like e.g. a heart, of a subject 118 with may be positioned.

Within the bore 106 of the magnet there is also a set of magnetic field gradient coils 110 forming a magnetic field gradient system which is used for acquisition of magnetic resonance data to spatially encode magnetic spins within the imaging zone 108 of the magnet 104. The magnetic field gradient coils 110 connected to a magnetic field gradient coil power supply 112. The magnetic field gradient coils 110 are intended to be representative. Typically, magnetic field gradient coils 110 contain three separate sets of coils for spatially encoding in three orthogonal spatial directions. A magnetic field gradient power supply supplies current to the magnetic field gradient coils. The current supplied to the magnetic field gradient coils 110 is controlled as a function of time and may be ramped or pulsed.

Adjacent to the imaging zone 108 is a radio-frequency coil 114, also referred to as radio-frequency antenna system, for manipulating the orientations of magnetic spins within the imaging zone 108 and for receiving radio transmissions from spins also within the imaging zone 108. The radio frequency coil 114 may contain multiple coil elements. The radio-frequency coil 114 is connected to a radio frequency transceiver 115. The radio-frequency coil 114 and radio frequency transceiver 115 may be replaced by separate transmit and receive coils and a separate transmitter and receiver. It is understood that the radio-frequency coil 114 and the radio frequency transceiver 115 are representative. The radio-frequency coil 114 is intended to also represent a dedicated transmit antenna and a dedicated receive antenna. Likewise, the transceiver 115 may also represent a separate transmitter and receivers. The radio-frequency coil 114 may also have multiple receive/transmit elements and the radio frequency transceiver 115 may have multiple receive/transmit channels.

The subject support 120 is attached to an optional actuator 122 that is able to move the subject support and the subject 118 through the imaging zone 108. In this way, a larger portion of the subject 118 or the entire subject 118 can be imaged. In particular the anatomical structure of interest 109 may be imaged. The transceiver 115, the magnetic field gradient coil power supply 112 and the actuator 122 are shown as being connected to a hardware interface 128 of a computer system provided by the medical apparatus 126.

The medical apparatus 126 may in addition comprise a catheter configured to be inserted into the subject 118 and moved to the anatomical structure of interest 109, in order to measure providing local blood oxygen saturation levels.

The computer 127 of Fig. 2 may in addition to the computer 127 shown in Fig. 1 comprise one or more pulse sequences 140 contained in the computer storage 134. The pulse sequences 140 are either instructions or data which can be converted into instructions which enable the processor 130 to acquire magnetic resonance data using the magnetic resonance imaging system 100. The computer storage 134 is further shown as containing magnetic resonance data 142 acquired by radio-frequency coil 114. From the magnetic resonance data 142 the graphical representation 144 may be reconstructed.

The computer memory 136 is shown as comprising a control module 160. The control module 160 contains computer executable code or instructions which enable the processor 130 to control the operation and function of the medical apparatus comprising the magnetic resonance imaging system 100 and the catheter. For instance, the control module 160 may work in conjunction with the pulse sequences 140 to acquire the various magnetic resonance imaging data 142. The computer memory 138 is shown as further containing a reconstruction module 162 which contains computer executable code or instructions which enable the processor 130 to control the operation and function of the magnetic resonance imaging system 100 to reconstruct the graphical representation 144, e.g. magnetic resonance images or a segmentation, from the acquired magnetic resonance imaging data 142.

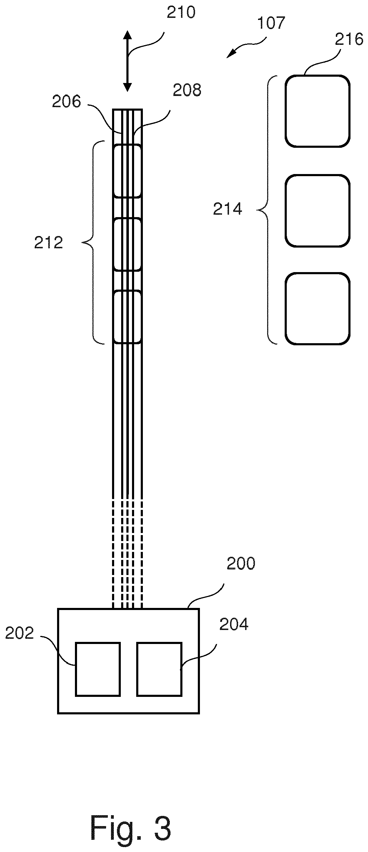

Fig. 3 illustrates an exemplary catheter 107. The catheter 107 may be configured to continuously measure local blood oxygen saturation levels using fiberoptic reflection spectrophotometry. Light 210 is emitted from a light source 202, like e.g. an LED, located in an oximetry unit 200 that is attached to the distal end of the catheter 107 through a first fiberoptic channel 206 comprised by the catheter 107 into the blood comprised by the anatomical structure of interest. Some of this light 210 is reflected back and received by a second fiberoptic channel 208 comprised by the catheter 107, which is read by a photodetector 204 located in the oximetry unit 200. The respective oximetry unit 200 may be provided in addition to the medical apparatus 126 or be comprised by the respective medical apparatus 126. The amount and spectrum of light 210 that is reflected by the blood is determined by the oxygen saturation of hemoglobin and thus provides a local blood oxygenation signal. This local blood oxygenation signal is processed by the medical apparatus 126, used for updating the spatial distribution of local blood oxygen saturation levels 146 and displayed on the display provided by the user interface 132, e.g. as a color coding indicating the local blood oxygen saturation levels.

Furthermore, the catheter 107 may comprise one or more coils 212 in order to determine the position and/or orientation of the tip of the catheter 107. The position of the tip of the catheter 107, at which the light 210 is emitted and received, corresponds to the position of the measurement of the local blood oxygen saturation level within the anatomical structure of interest 109. The one or more coils 212 may detect voltage fluctuations induced due to moving the catheter 107 within a spatially varying magnetic field. The spatially varying magnetic field may be provided by an electro-magnetic tracking system 214 comprising a plurality of coils 216 generating each generating a varying magnetic field which combine to the spatially varying magnetic field. According to alternative embodiments, the spatially varying magnetic field may be generated by the magnetic resonance imaging system 100.

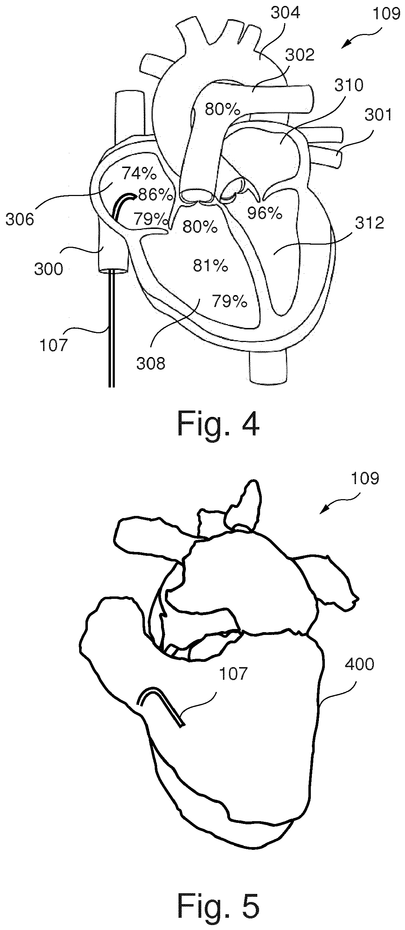

Fig. 4 depicts a schematic diagram of an anatomical structure of interest 109 in form of a heart illustrating exemplary local blood oxygen saturation levels within the heart 109. Here, the values of the local blood oxygen saturation levels are provided in form of percentage values. The local blood oxygen saturation levels may be measured by the catheter 107 moved through the heart 109 e.g. in the vena cava 300, pulmonary vein 301, pulmonary artery 302, aorta 304, right atrium 306, right ventricle 308, left atrium 310, and/or left ventricle 312. Typical values of the local blood oxygen saturation levels are depicted with the exception of an elevated value of 86% in the right atrium 306 indicating a left-to-right shunt in the right atrium 306. Thus, FIG. 4 illustrates a typical situation of oxygenation in case of a left-to-right shunt in the right atrium 306. When a catheter 107 is navigated from the vena cava through the right atrium 306 and right ventricle 308 to the pulmonary artery 302, both, continuous oximetry measurements and 3D position measurements, e.g. by MR-tracking, may be performed. The data may be used to fill a 3D oxygenation dataset providing the spatial distribution of the local blood oxygen saturation levels with data points wherever the catheter tip, i.e. the position of measurement, has been located so far.

Fig. 5 depicts a graphical representation of the heart 109 in form of a segmentation comprising a plurality of segments 400. The segments 400 may be showing in form of mesh structure rendering spatial contours of a heart 109 within which the catheter 107 is moved forward.

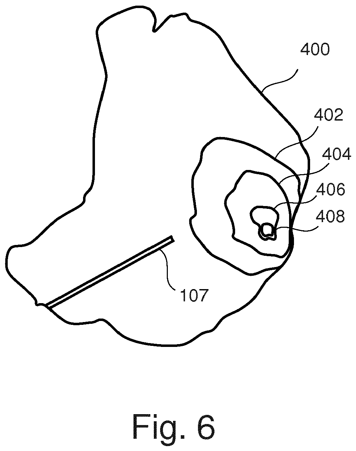

Fig. 6 illustrates a segment 400 representing the endocardium in which a color-coding in form of regions with different colors 402 to 408 is projected onto contours of the right atrium 306, i.e. the endocardium. The color-coding 402 to 408 indicates the local blood oxygen saturation levels measured and/or interpolated or extrapolated in close vicinity to the respective contours of the segment 400, i.e. on the inner surfaces of the endocardium. These endocardial surface of a specific chamber, e.g. the right atrium 306, maybe color-coded using oximetry extrapolated from measurements inside that chamber. Effectively, this approach may only color-code 402 to 408 the contours of the segment 400 of the anatomical structure of interest, e.g. the walls of the heart. In other words, a spatial distribution of the local blood oxygen saturation levels on the inners surfaces of the anatomical structure of interest may be provided. This mode may have the beneficial effect that 3D data may be reduced to a projection on 2D surface of the segment 400 which are intuitively perceived. In addition, orifices of cardiac shunts have to lie in this surface. Thus, considering the spatial distribution of the local blood oxygen saturation levels depicted by the color-coding 402 to 408 on this surface may allow for efficiently determining the presence, position, and/or form of cardiac shunts. The color-coding 402 to 408 depicted in Fig. 6 hints to a shunt to a chamber with e.g. more highly oxygenated blood the region 408.

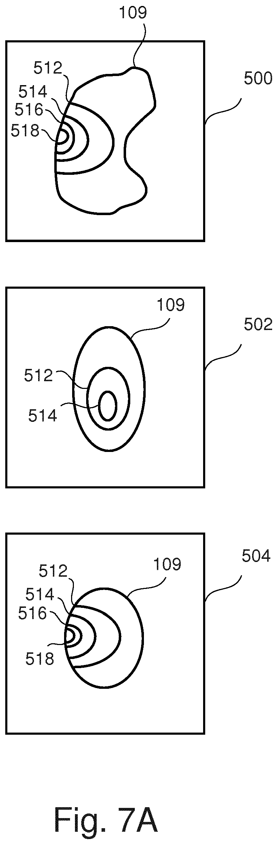

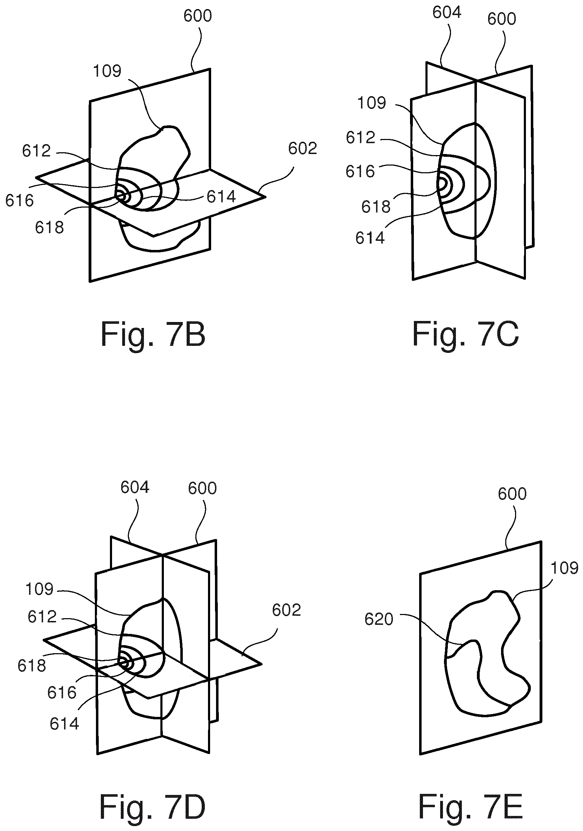

Figs. 7 illustrate graphical representations comprising one or magnetic resonance images 500 to 504 and 600 to 604.The oximetry data is interpolated and/or extrapolated throughout the planes, orthogonal planes in the case depicted, represented by the two-dimensional magnetic resonance images 500 to 504 and 600 to 604 and indicated as a color-coding 512 to 518 and 612 to 618 which is projected onto these planes. This may allow for a manual exploration of the 3D data, e.g. by moving a common center point or center line at which the planes intersect e.g. using a mouse. In an alternative mode, the center point or center line may automatically follow the catheter 107 in real time. In order to allow for a simpler visualization, only one or two of the planes may be used, while the others are switched off.

Fig. 7B illustrates a graphical representation comprising three magnetic resonance images 500 to 504. The three magnetic resonance images 500 to 504 correspond to three orthogonal cross-sections through the anatomical structure of interest 109 also depicted in Fig. 6. In addition to the contours of the cross-section of the anatomical structure of interest 109, the spatial distribution of the local blood oxygen saturation levels is depicted by a color-coding 512 to 518 in form of regions with different colors. In addition, the position of the tip of the catheter 107 be indicated in the magnetic resonance images 500 to 504. Fig. 7B illustrates a graphical representation comprising two magnetic resonance images 600, 602 shown together and representing two orthogonal cross-sections through the anatomical structure of interest 109 also depicted in Fig. 6. Again, spatial distribution of the local blood oxygen saturation levels is depicted by a color-coding 612 to 618 in form of regions with different colors. Fig. 7C illustrates a graphical representation comprising a different combination of two magnetic resonance images 600, 604 representing a different combination of orthogonal cross-sections through the anatomical structure of interest 109. Fig. 7D illustrates a combination of the graphical representations shown in Fig. 7B and FIG. 7C comprising three magnetic resonance images 600 to 604 representing three orthogonal cross-sections through the anatomical structure of interest 109. Finally, Fig 7E illustrates a graphical representation comprising a single magnetic resonance image 600 showing a cross-section through the anatomical structure of interest 109 and a track 620 of the tip of the catheter 107 which has been moved within the cross-sectional plane. The track 620 may be depicted color-coded indicating the local blood oxygen saturation levels measured along the track 620.

The graphical representations shown in Fig 6 and FIGS. 7 may e.g. be displayed together on the display or the display may switch from one representation to another. The displayed visualizations may generally be updated by newly acquired data in real-time during the catheterization procedure. An exemplary use case may for example be detecting and finding an exact location of an orifice of a shunt. An unexpected local change of blood oxygenation may be a first indication of a shunt feeding that region. If such a change is detected, it may e.g. first be switched to the first representation mode depicted in FIGS. 7 and the catheter 107 roved locally. Such a local gradient of blood oxygenation may be found which points to the shunt orifice 518, 618. Then the catheter 107 maybe moved towards the suspected orifice 518, 618 following the gradient of blood oxygenation 512 to 518, 612 to 618. When the catheter 107 approaches the cardiac wall, the visualization may be switched manually or automatically the second representation mode illustrated in Fig. 6. This may allow to move the catheter 107 along the wall to find the exact position of the orifice 408.

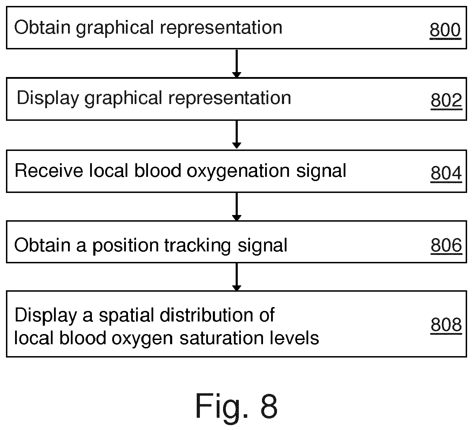

Fig. 8 shows a flowchart which illustrates a method of operating the medical apparatus. In step 800 a graphical representation of magnetic resonance data of an anatomical structure of interest is obtained. The graphical representation is e.g. obtained using magnetic resonance data acquired at the beginning of the catheterization. In step 802, the graphical representation is displayed on the display. In step 804, a local blood oxygenation signal is received from a catheter providing local blood oxygen saturation levels measured by the catheter within the anatomical structure of interest. In step 806, a position tracking signal of the catheter is obtained tracking positions of the measurements of the local blood oxygen saturation levels within the anatomical structure of interest. Steps 804 and 806 may e.g. be performed simultaneously and constantly retreaded. In step 808 a spatial distribution of local blood oxygen saturation levels is displayed within the graphical representation displayed on the display using the measured local blood oxygen saturation levels from step 804 and the tracked positions of the measurements from step 806. In addition, the position and/or orientation of the catheter, in particular the catheter tip, may be indicated in the graphical representation displayed on the display. The spatial distribution of local blood oxygen saturation levels may be updated, whenever the steps 804 and 806 are repeated.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a 'circuit', 'module' or 'system'. Furthermore, aspects of the present invention may take the form of a computer program product embodied in one or more computer readable medium(s) having computer executable code embodied thereon.

Any combination of one or more computer readable medium(s) maybe utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

A 'computer memory' or 'memory' is an example of a computer-readable storage medium. A computer memory is any memory which is directly accessible to a processor. A 'computer storage' or 'storage' is a further example of a computer-readable storage medium. A computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising 'a processor' should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the 'C' programming language or similar programming languages and compiled into machine executable instructions. In some instances, the computer executable code may be in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection maybe made to an external computer (for example, through the Internet using an Internet Service Provider).

Aspects of the present invention are described with reference to flowchart illustrations and/or block diagrams of methods, apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device'. A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word 'comprising' does not exclude other elements or steps, and the indefinite article 'a' or 'an' does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: magnetic resonance imaging system
- 104: main magnet
- 106: bore of magnet
- 107: catheter
- 108: imaging zone
- 109: anatomical structure of interest
- 110: magnetic field gradient coil
- 112: magnetic field gradient coil power supply
- 114: radio-frequency coil
- 115: transceiver
- 118: subject
- 120: subject support
- 122: actuator
- 126: medical apparatus
- 127: computer
- 128: hardware interface
- 130: processor
- 132: user interface
- 134: computer storage
- 136: computer memory
- 140: pulse sequences
- 142: magnetic resonance data
- 144: graphical representation
- 146: spatial distribution of local blood oxygen saturation levels
- 148: position and/or orientation data
- 160: control module
- 161: control module
- 162: reconstruction module
- 164: spatial distribution generating module
- 166: catheter position generating module
- 168: display control module
- 200: oximetry unit
- 202: light source
- 204: photodetector
- 206: fiberoptic channel
- 208: fiberoptic channel
- 210: light
- 212: coils
- 214: electro-magnetic tracking system
- 216: coils
- 300: vena cava
- 301: pulmonary vein
- 302: pulmonary artery
- 304: aorta
- 306: right atrium
- 308: right ventricle
- 310: left atrium
- 312: left ventricle
- 400: segment
- 402: color-coding
- 404: color-coding
- 406: color-coding
- 408: color-coding
- 500: magnetic resonance image
- 502: magnetic resonance image
- 504: magnetic resonance image
- 512: color-coding
- 514: color-coding
- 516: color-coding
- 518: color-coding
- 600: magnetic resonance image
- 602: magnetic resonance image
- 604: magnetic resonance image
- 612: color-coding
- 614: color-coding
- 616: color-coding
- 618: color-coding
- 620: track

## Claims

1. A medical apparatus (126) comprising:
a display (132);
a processor (130) for controlling the medical apparatus (126);
a memory (136) storing machine executable instructions (160-168) for execution by the processor (130);
wherein execution of the instructions (160-168) causes the processor (130) to control the medical apparatus (126) to:
- obtain a graphical representation (144) of magnetic resonance data (142) of an anatomical structure of interest (109);
- display the graphical representation (144) on the display (132);
- receive a local blood oxygenation signal from a catheter (107) providing local blood oxygen saturation levels measured by the catheter (107) within the anatomical structure of interest (109);
- obtain a position tracking signal of the catheter (107) tracking positions (148) of the measurements of the local blood oxygen saturation levels within the anatomical structure of interest (109);
- display a spatial distribution (146, 402-408, 512-518, 612-618) of local blood oxygen saturation levels within the graphical representation (144) displayed on the display (132) using the measured local blood oxygen saturation levels and the tracked positions (148) of the measurements.

2. The medical apparatus (126) of claim 1, the graphical representation (144) comprises a segmentation depicting one or more segments (400) of the anatomical structure of interest (109) and/or one or more magnetic resonance images (500-504, 600-604), each magnetic resonance image depicting a cross-section through the anatomical structure of interest (109).

3. The medical apparatus (126) of any of claims 1 or 2, the anatomical structure of interest (109) comprising one or more blood vessels (300-304), left atrium (310) of a heart, right atrium (306) of the heart, left ventricle (312) of the heart and/or right ventricle (308) of the heart.

4. The medical apparatus (126) of any of claims 1 to 3, the displaying of the spatial distribution (146, 402-408, 512-518, 612-618) of the local blood oxygen saturation levels comprising:
interpolating or extrapolating additional local blood oxygen saturation levels at positions within the anatomical structure of interest (109), for which no measured local blood oxygen saturation levels are provided by the catheter (107), using the measured local blood oxygen saturation levels and the tracked positions (148) of the measurements, the displayed spatial distribution (146, 402-408, 512-518, 612-618) of the local blood oxygen saturation levels comprising the additional local blood oxygen saturation levels.

5. The medical apparatus (126) of any of claims 4, for the interpolating or the extrapolating of each of the additional local blood oxygen saturation levels taking into account only those measured local blood oxygen saturation levels at positions which are geometrically connectable with the position at which the respective additional local blood oxygen saturation level is to be interpolated or extrapolated by a line such that the line is not crossing any spatial contour of the anatomical structure of interest (109) and
which have a distance along the respective line from the position at which the respective additional local blood oxygen saturation level is to be interpolated or extrapolated that is smaller than a predefined maximum distance.

6. The medical apparatus (126) of any of claims 4 to 5, in a first display mode, the displayed graphical representation (144) comprising one or more of the two-dimensional images (500-504, 600-604) generated using multiplanar reformation, the spatial distribution (146, 402-408, 512-518, 612-618) of the local blood oxygen saturation levels being interpolated or extrapolated in the respective one or more two-dimensional images (500-504, 600-604).

7. The medical apparatus (126) of any of claim 6, the cross-sections depicted by the one or more displayed two-dimensional images (500-504, 600-604) automatically following the positions (148) tracked by the tracking signal of the catheter (107).

8. The medical apparatus (126) of any of claims 4 to 7, in a second display mode, the displayed graphical representation (144) comprising the segmentation depicting one or more segments (400) of the anatomical structure and the displayed spatial distribution (146, 402-408, 512-518, 612-618) of the local blood oxygen saturation levels comprising additional local blood oxygen saturation levels extrapolated on one or more spatial contours of the one or more segments (400).

9. The medical apparatus (126) of any of claims 4 to 8, continuously updating the interpolation or extrapolation of the additional local blood oxygen saturation levels comprised by the displayed spatial distribution (146, 402-408, 512-518, 612-618) of local blood oxygen saturation levels using the measured local blood oxygen saturation level currently received.

10. The medical apparatus (126) of any of claims 1 to 3, the spatial distribution (146, 402-408, 512-518, 612-618) of the local blood oxygen saturation levels being displayed in form of a line (620) following the tracked positions (148) of the measurements of the local blood oxygen saturation levels within anatomic structure of interest (109) depicting the local blood oxygen saturation levels measured at the respective tracked positions (148).

11. The medical apparatus (126) of any of the previous claims, the displaying of the spatial distribution (146, 402-408, 512-518, 612-618) of the local blood oxygen saturation levels further providing an alarm signal if one of the local blood oxygen saturation levels comprised by the spatial distribution (146, 402-408, 512-518, 612-618) of local blood oxygen saturation levels or if a spatial and/or temporal gradient of the local blood oxygen saturation levels exceeds a threshold.

12. The medical apparatus (126) of any of the previous claims, further comprising a magnetic resonance imaging system (100) for acquiring magnetic resonance data (142) from an imaging zone (108),
wherein execution of the instructions (161-168) further causes the processor (130) to control the magnetic resonance imaging system (100) to:
- acquire magnetic resonance data (142) of the anatomical structure of interest (109) within the imaging zone (108);
- reconstruct the graphical representation (144) using the acquired magnetic resonance data (142);
- provide the graphical representation (144).

13. The medical apparatus (126) of any of the previous claims, further comprising the catheter (107),
wherein execution of the instructions (161-168) further causes the processor (130) to control the medical apparatus (126) to:
- provide the local blood oxygenation signal;
- provide the position tracking signal of the catheter (107).

14. A computer program product comprising machine executable instructions (160-168) for execution by a processor (130) controlling a medical apparatus (126), the medical apparatus (126) further comprising:
- a display (132);
- a memory (136) for storing the machine executable instructions (160-168) for execution by the processor (130);
wherein execution of the instructions (160-168) causes the processor (130) to control the medical apparatus (126) to:
- obtain a graphical representation (144) of magnetic resonance data (142) of an anatomical structure of interest (109);
- display the graphical representation (144) on the display (132);
- receive a local blood oxygenation signal from a catheter (107) providing local blood oxygen saturation levels measured by the catheter (107) within the anatomical structure of interest (109);
- obtain a position tracking signal of the catheter (107) tracking positions (148) of the measurements of the local blood oxygen saturation levels within the anatomical structure of interest (109);
- display a spatial distribution (146, 402-408, 512-518, 612-618) of local blood oxygen saturation levels within the graphical representation (144) displayed on the display (132) using the measured local blood oxygen saturation levels and the tracked positions (148) of the measurements.

15. A method for operating a medical apparatus (126) comprising:
- a display (132);
- a processor (130) for controlling the medical apparatus (126);
- a memory (136) storing machine executable instructions (160-168) for execution by the processor (130);
wherein execution of the instructions (160-168) causes the processor (130) to control the medical apparatus (126) to execute the method comprising:
- obtaining a graphical representation (144) of magnetic resonance data (142) of an anatomical structure of interest (109);
- displaying the graphical representation (144) on the display (132);
- receiving a local blood oxygenation signal from a catheter (107) providing local blood oxygen saturation levels measured by the catheter (107) within the anatomical structure of interest (109);
- obtaining a position tracking signal of the catheter (107) tracking positions (148) of the measurements of the local blood oxygen saturation levels within the anatomical structure of interest (109);
- displaying a spatial distribution (146, 402-408, 512-518, 612-618) of local blood oxygen saturation levels within the graphical representation (144) displayed on the display (132) using the measured local blood oxygen saturation levels and the tracked positions (148) of the measurements.
